# EUROPÄISCHE PATENTANMELDUNG

(11) **EP 3 228 692 A2**
(43) Veröffentlichungstag der Anmeldung: **11.10.2017**
(21) Anmeldenummer: 17164113.7
(22) Anmeldetag: 31.03.2017
(51) Int. Cl.: C12M 1/107, C12M 1/00, C12P 5/00

(54) **VERFAHREN UND VORRICHTUNG ZUR AUFBEREITUNG VON BIOMASSE**

(30) Priorität: 04.04.2016 AT 502722016
(71) Anmelder: SFL Technologies GmbH, 8152 Stallhofen (AT)
(72) Erfinder: Höllwart, Johann, 8042 Graz (AT); Müller, Mario, 8047 Kainbach bei Graz (AT)
(74) Vertreter: Wirnsberger & Lerchbaum Patentanwälte OG

(57) **Zusammenfassung**

Die Erfindung betrifft eine Vorrichtung (1) zur Aufbereitung von Biomasse für einen Fermenter, umfassend zumindest ein Behältnis (3), zumindest eine Zuleitung (4) und zumindest eine Ableitung (5), wobei Biomasse über die Zuleitung (4) in das zumindest eine Behältnis (3) führbar und über die zumindest eine Ableitung (5) aus diesem abführbar ist. Erfindungsgemäß ist vorgesehen, dass das zumindest eine Behältnis (3) mit zumindest einem Dampfeinlass (6) und zumindest einem Dampfauslass (7) ausgebildet ist und zumindest eine nebengeordnete Dampfzirkulationseinheit (8) vorgesehen ist, welche über den zumindest einen Dampfeinlass (6) und den zumindest einen Dampfauslass (7) mit dem Behältnis (3) in Verbindung steht und über welche Dampf im Behältnis zirkulierbar ist, um die Biomasse im Behältnis (3) unter erhöhtem Druck und bei erhöhter Temperatur zu behandeln, und dass dem Behältnis (3) stromabwärts ein Entspannungsbehälter (9) nachgestaltet ist, in welchem die im Behältnis (3) behandelte Biomasse gegen Atmosphärendruck entspannbar ist.

Des Weiteren betrifft die Erfindung ein Verfahren zur Aufbereitung von Biomasse mit einer entsprechenden Vorrichtung (1), eine mit dem Verfahren hergestellte fließfähige Masse sowie eine Fermenteranordnung (2).

## Beschreibung

Die Erfindung betrifft eine Vorrichtung zur Aufbereitung von Biomasse für einen Fermenter, umfassend zumindest ein Behältnis, zumindest eine Zuleitung und zumindest eine Ableitung, wobei Biomasse über die Zuleitung in das zumindest eine Behältnis führbar und über die zumindest eine Ableitung aus diesem abführbar ist.

Des Weiteren betrifft die Erfindung ein Verfahren zur Aufbereitung von Biomasse für einen Fermenter mit einer Vorrichtung der vorstehenden Art.

Des Weiteren betrifft die Erfindung eine fließfähige Masse.

Schließlich betrifft die Erfindung eine Fermenteranordnung, umfassend mehrere in Serie geschaltete Fermenter zur Aufbereitung von Biomasse, insbesondere Abfällen und/oder Müll aus Biomasse.

Ein beträchtlicher Teil des heute anfallenden Abfalls oder Mülls ist biologischer Natur bzw. Herkunft. Bereits in kleineren Großstädten können jährlich mehrere zehntausend Tonnen an Biomüll anfallen.

Wie bei anderen Abfällen ist es üblich, auch Abfälle biologischer Herkunft nach Möglichkeit zu verwerten. Für biologische Abfälle kommt häufig eine Verwertung durch Fermentation bzw. Abbau der festen biologischen Abfälle zu Gasen infrage. Hierfür wird üblicherweise in einer konventionellen Biogasanlage ein Rohsubstrat mit dem biologischen Müll zunächst in einem Hauptfermenter fermentiert und anschließend einem Nachfermenter zugeführt, in dem noch nicht umgesetzte Bestandteile bestmöglich ebenso zu Biogas umgewandelt werden sollen. Das in den Fermentern entstehende Biogas wird einem Blockheizkraftwerk zugeleitet, wo das Biogas zur Gewinnung von elektrischer Energie oder Wärmeenergie eingesetzt werden kann.

Wenngleich sich eine Gewinnung von Biogas aus biologischen Abfällen punktuell etabliert hat, gehen mit entsprechenden Verfahren neben den Vorteilen auch Nachteile einher. Ein Nachteil herkömmlicher Verfahren besteht darin, dass der Aufschluss der biologischen Materialen in den Fermentern nicht vollständig ist bzw. relativ hohe Anteile an nicht von Bakterien umsetzbaren Stoffen als Rest verbleiben, beispielsweise Lignocellulose und entsprechende Derivate. Zudem sind ungeachtet der unvollständigen Umsetzung lange Verweilzeiten in Fermentern erforderlich. Ein anderer Nachteil besteht darin, dass das Biogas zwar Methan für die Verbrennung sowie neben Wasserstoff und Sauerstoff auch Kohlenstoffdioxid und Stickstoff als geruchsneutrale Gase enthält, darüber hinaus aber auch in der Regel einen merklichen Anteil an Schwefelwasserstoff und anderen geruchsbelästigenden Substanzen vorliegt. Insbesondere Schwefelwasserstoff führt bereits in geringen Konzentrationen zu deutlichen Geruchsbelästigungen. Ein weiterer Nachteil besteht darin, dass die Energiedichte des erzeugten und später in einem Blockheizkraftwerk umgesetzten Biogases aufgrund des gasförmigen Aggregatzustandes relativ gering ist.

Aufgabe der Erfindung ist es, eine Vorrichtung der eingangsgenannten Art anzugeben, mit welcher bestehende Fermentationsanlagen zur Erzeugung von Biogas nachgerüstet werden können und welche die vorstehend erläuterten Nachteile zumindest teilweise vermindert.

Eine weitere Aufgabe besteht darin, ein Verfahren der eingangs genannten Art entsprechend zu verbessern.

Ein weiteres Ziel der Erfindung ist es, eine fermentierbare Masse bereitzustellen, die eine hohe Energiedichte aufweist.

Schließlich ist es ein Ziel der Erfindung, eine Fermenteranordnung der eingangs genannten Art derart weiterzubilden, dass eine kurze Verweilzeit zur Erzeugung von Biogas in einzelnen Fermentern gegeben ist.

Die Aufgabe wird mit einer Vorrichtung der eingangs genannten Art gelöst, wobei das zumindest eine Behältnis mit zumindest einem Dampfeinlass und zumindest einem Dampfauslass ausgebildet ist und zumindest eine nebengeordnete Dampfzirkulationseinheit vorgesehen ist, welche über den zumindest einen Dampfeinlass und den zumindest einen Dampfauslass mit dem Behältnis in Verbindung steht und über welche Dampf im Behältnis zirkulierbar ist, um die Biomasse im Behältnis unter erhöhtem Druck und bei erhöhter Temperatur zu behandeln, und dass dem Behältnis stromabwärts ein Entspannungsbehälter nachgeschaltet ist, in welchem die im Behältnis behandelte Biomasse gegen Atmosphärendruck entspannbar ist.

Ein mit einer erfindungsgemäßen Vorrichtung erzielter Vorteil ist insbesondere darin zu sehen, dass durch das vorgesehene zumindest eine Behältnis samt nebengeordneter Dampfzirkulationseinheit eine Behandlung der aufzubereitenden Biomasse mit Dampf bei erhöhter Temperatur und erhöhtem Druck erfolgen kann. Dadurch können insbesondere durch Bakterien nicht oder nur in sehr langen Zeiträumen umsetzbare biologische Materialen so weit aufgeschlossen werden, dass nachfolgend eine rasche und im Idealfall vollständige Fermentation möglich ist. Durch den dem Behältnis nachgeordneten Entspannungsbehälter ergibt sich zudem der Vorteil, dass im Anschluss an die im Behältnis erfolgende Thermohydrolyse bei der Entspannung freiwerdende Energie wieder gezielt in den Energiekreislauf eingebunden werden kann. Insbesondere kann die freiwerdende Energie dazu genutzt werden, im Kreislauf befindliche Massen vorzuwärmen, beispielsweise eine dem zumindest einen Behältnis zugeführte Biomasse. Dies erlaubt eine energieeffiziente Verfahrensführung.

Bevorzugt sind mehrere Behältnisse vorgesehen. Die Behältnisse sind dabei bevorzugt gleich aufgebaut bzw. gleichwertig. Jedes der Behältnisse wird dann über eine Zuleitung gespeist, wobei die Zuleitungen vorzugsweise so gelegt sind, dass von einem Biomassereservoir zunächst eine einzelne Versorgungsleitung wegläuft, die anschließend in einzelne Zuleitungen für die jeweiligen Behältnisse übergeht. Dadurch ist es möglich, mehrere Behältnisse zu beschicken, wobei eine Beschickung simultan oder sequenziell erfolgen kann.

Wenn mehrere Behältnisse vorgesehen sind, sind diese zum Zwecke eines hohen Durchsatzes zweckmäßigerweise parallel geschaltet. Die Anzahl der Behältnisse richtet sich dabei nach der Anlagengröße bzw. einer pro Zeiteinheit aufzuarbeitenden Biomasse. Des Weiteren kann einem Durchsatz auch durch geeignete Skalierung der Behältnisse ausreichend Rechnung getragen werden.

Die Dampfzirkulationseinheit umfasst mit Vorteil einen ersten Wärmetauscher, insbesondere einen Rohrbündelwärmetauscher. Dadurch ist es möglich, dass im Kreislauf zwischen Dampfzirkulationseinheit und Behältnis geführter Dampf zunächst die in das Behältnis eingeführte Biomasse erwärmt und anschließend im Rohrbündelwärmetauscher wieder vorgewärmt wird. Die Vorwärmung im Rohrbündeltauscher kann dabei beispielsweise über die Abwärme von Motoren eines Blockheizkraftwerkes erfolgen, das einer Fermenteranordnung nachgeschaltet ist. Dadurch wird die vom Blockheizkraftwerk generierte Abwärme effizient genutzt, was zu einer hohen Energieeffizienz eines entsprechenden Verfahrens beiträgt. Möglich ist es alternativ oder ergänzend aber auch, überschüssige Regelenergie aus dem Blockheizkraftwerk für die Vorwärmung zu nutzen. Beispielsweise kann mit dieser Regelenergie Dampf für die Vorwärmung erzeugt werden.

In Bezug auf eine Energieeffizienz ist es des Weiteren bevorzugt, dass zumindest ein zweiter Wärmetauscher vorgesehen ist, welcher dem Entspannungsbehälter nachgeschaltet ist und mit der welcher mit der Zuleitung in Wirkverbindung steht, sodass die Biomasse vor deren Zuführung zum Behältnis durch Wärmetausch vorwärmbar ist. Dadurch kann im Entspannungsbehälter frei werdende Energie selektiv zur Vorwärmung der im zumindest einen Behältnis zu behandelnden Biomasse genutzt werden. Insbesondere kann die so zugeführte Biomasse beispielsweise auf eine Temperatur von etwa 80 °C bis 90 °C gebracht werden.

Für die gewünschte Umsetzung der Biomasse in zumindest einem Behältnis kann vorgesehen sein, dass das Behältnis für Temperaturen von zumindest 120 °C, vorzugsweise zumindest 150 °C, insbesondere zumindest 180 °C, ausgebildet ist. Damit auch hohe Drücke unproblematisch sind, kann das Behältnis weiter für Drücke von bis zu 5 bar, vorzugsweise bis zu 10 bar, besonders bevorzugt bis zu 15 bar insbesondere bis zu 20 bar, druckresistent ausgebildet sein.

Eine erfindungsgemäße Vorrichtung kann grundsätzlich mit beliebiger Größe und stationär einsetzbar ausgebildet sein. Es ist für verschiedene Anwendungen jedoch von Vorteil, wenn die Vorrichtung transportabel, insbesondere mit einem LKW transportierbar, ausgebildet ist. Hierfür können ein oder mehrere Container vorgesehen sein, welche die Vorrichtung zumindest weitgehend außenseitig abschließen. Insbesondere ISO-Container eignen sich dafür. Die Vorrichtung kann dann werkseitig in einem oder mehreren Containern platziert werden, wobei an dem oder den Containern außenseitig Anschlusspunkte zur Einbindung in eine bestehende Infrastruktur gegeben sind. Durch einen Container weitgehend abgeschlossen bedeutet dabei, dass durchaus außenseitig noch einzelne Rohre verlaufen können, so lange dies einen Transport des Containers insbesondere auf einem LKW oder Frachtschiff nicht behindert. Eine entsprechende Ausbildung mit einer Hülle eines Containers, insbesondere eines ISO-Containers, oder gegebenenfalls mehrerer entsprechender Container erlaubt vor allem ein Nachrüsten bestehender Anlagen und einen raschen Transport sowie eine schnelle Einbindung in bestehende Infrastrukturen. Darüber hinaus ergibt sich auch der Vorteil, dass die Vorrichtung bei mangelndem Bedarf umgekehrt rasch aus der bestehenden Infrastruktur herausgelöst werden kann.

Die verfahrensmäßige Aufgabe der Erfindung wird gelöst, wenn bei einem Verfahren der eingangs genannten Art in zumindest einem Behältnis Dampf kontinuierlich zirkuliert und die Biomasse für eine vorbestimmte Zeit in Behältnis gehalten und anschließend einem Entspannungsbehälter zugeführt wird.

Bei einer entsprechenden Verfahrensführung wird die Biomasse zumindest einem Behältnis zugeführt und verweilt zur Umsetzung eine bestimmte Zeitspanne in diesem. Dampf wird vorzugsweise im Gegenstrom durch das Behältnis zirkuliert, insbesondere kontinuierlich zirkuliert, sodass insbesondere von Bakterien nicht umsetzbare Biomasse bei erhöhter Temperatur und erhöhtem Druck so weit aufgeschlossen wird, dass diese nachfolgend auch einer Fermentation zugänglich ist. Der entsprechende Verfahrensschritt kann dabei bei herkömmlichen Anlagen bereits vor einem Hauptfermenter stattfinden. Möglich ist es jedoch auch, dass ein entsprechendes Verfahren bei einer bestehenden Fermentationsanlage zwischen einem Hauptfermenter und einem Nachfermenter durchgeführt wird.

Um einen möglichst hohen Durchsatz an Biomasse zu erreichen, ist es zweckmäßig, dass die Biomasse in mehreren, vorzugsweise parallel geschalteten Behältnissen behandelt wird. Grundsätzlich können die Behältnisse in diesem Fall unabhängig voneinander betrieben werden. Es kann jedoch insbesondere vorgesehen sein, dass die Biomasse in den Behältnissen einem Zyklus mit Befüllung, Aufheizen auf eine vorbestimmte Temperatur und Halten bei dieser Temperatur unterworfen wird, wobei die Zyklen in den Behältnissen zeitversetzt ablaufen können. Dies ermöglicht eine Abstimmung der kontinuierlichen Dampfzirkulation einerseits auf die semikontinuierliche Zuführung von Biomasse zu einzelnen Behältnissen andererseits. Sind beispielsweise vier Behältnisse vorgesehen, ist eines der Behältnisse in einer Aufheizphase, in welcher die meiste Energie benötigt wird, zwei Behältnisse befinden sich im Haltemodus und ein weiteres Behältnis ist im Stadium des Entleerens in den Entspannungsbehälter. Durch eine entsprechend getaktetes Verfahren kann ohne Weiteres die erforderliche Energie für das Aufheizen bereitgestellt werden. Darüber hinaus ist ein regelmäßiger Betrieb des Entspannungsbehälters und damit, im Zusammenhang mit der vorgesehen Energierückgewinnung durch einen Wärmetauscher, einer Energiebereitstellung gegeben.

Zweckmäßig ist es, um eine Aufheiztemperatur möglichst gering zu halten, wenn die Biomasse vor der Zuführung zum Behältnis erwärmt wird. Hierfür kann insbesondere im Entspannungsbehälter freiwerdende Energie genutzt werden. Die freiwerdende Energie kann beispielsweise über einen Wärmetauscher, der dem Entspannungsbehälter nachgeschaltet ist, entzogen und zur Vorwärmung von Biomasse bereitgestellt werden.

Die Aufbereitung der Biomasse im Behältnis sollte so erfolgen, dass das entstehende Substrat in einem nachgeschalteten Fermenter möglichst vollständig umgesetzt werden kann. Hierfür ist es zweckmäßig, wenn die Biomasse im Behältnis bei einer Temperatur von 120 °C bis 300 °C, vorzugsweise 165 °C bis 220 °C, und einem Druck von zumindest 5 bar, vorzugsweise 7 bar bis 17 bar, behandelt wird.

Bei einer Vorgehensweise entsprechend dem vorstehend dargelegten Verfahren hat sich gezeigt, dass die aus dem Entspannungsbehälter abnehmbare, einer Thermohydrolyse unterworfene und so voraufbereitete Biomasse als fließfähige Masse vorliegt. Im Idealfall können ausschließlich flüssige Anteile vorliegen, die aus dem Entspannungsbehälter abzweigbare Masse ist aber auch bei Feststoffanteilen fließfähig. Dies stellt einen entscheidenden Vorteil dar, weil die entsprechende Masse mit geringem Volumen zwischengelagert werden kann. Mit anderen Worten: Es ist nicht erforderlich, dass die aus dem Entspannungsbehälter abgezweigte Masse sofort einer weiteren Fermentation zugeführt wird. Dies ergibt völlig neue Möglichkeiten in Bezug auf die Aufbereitung von Biomüll, weil die Voraufbereitung bzw. Thermohydrolyse auch örtlich entkoppelt von der eigentlichen Fermentation stattfinden kann. Dementsprechend stellt eine derart hergestellte fließfähige Masse einen weiteren Aspekt der Erfindung dar. Neben der Lager- und Transportfähigkeit weist eine so hergestellte Masse den zusätzlichen Vorteil auf, dass diese steril ist. Aufgrund der im Vergleich mit Gas aus Biomasse hohen Dichte kann die Flüssigkeit auch als temporärer Energiespeicher angesehen werden.

Ein erfindungsgemäßes Verfahren kann bei bestehenden Fermentationsanlagen angewendet werden. Dabei kann die Thermohydrolyse samt Entspannung vor einem Hauptfermenter oder gegebenenfalls auch zwischen Hauptfermenter und nachgeschaltetem Nachfermenter stattfinden. Dementsprechend umfasst eine Fermenteranordnung vorzugsweise einen Hauptfermenter und einen nachgestalteten Nachfermenter, wobei eine erfindungsgemäße Vorrichtung dem Hauptfermenter vorgeschaltet oder stromabwärts vor dem Nachfermenter dem Hauptfermenter nachgeschaltet ist.

Das weitere Ziel der Erfindung wird durch eine Fermenteranordnung der eingangs genannten Art erreicht, wobei ein Vorfermenter und ein Hauptfermenter vorgesehen sind und im und/oder am Vorfermenter und Hauptfermenter Mittel zur Umwälzung der aufzubereitenden Biomasse vorgesehen sind.

Ein mit einer erfindungsgemäßen Fermentanordnung erzielter Vorteil ist insbesondere darin zu sehen, dass aufgrund der vorgesehen Mittel zur Umwälzung der Biomasse diese sehr kurz in den Fermentern gehalten werden kann, um die erforderliche Gasproduktion sicherzustellen. Im Gegensatz zum Stand der Technik reduziert sich die erforderliche Zeit für eine möglichst vollständige Umsetzung auf wenige Tage anstelle von Wochen.

Im Übrigen kann im Rahmen der Erfindung eine Fermenteranordnung mit einer erfindungsgemäßen Vorrichtung und zumindest einem Fermenter zweckmäßig sein, wobei der zumindest eine Fermenter mit einer Wasserstoffquelle in Verbindung steht und/oder mit dieser in Verbindung bringbar ist, sodass Wasserstoff in den zumindest einen Fermenter einbringbar ist. Durch die zusätzliche Einbringung von Wasserstoff lässt sich die Ausbeute an Methan aus dem Fermenter signifikant steigern. Während ohne zusätzliche Wasserstoffzufuhr in den Fermenter eine Ausbeute in Bezug auf die maximal erreichbar Menge an Methan bei 65 % liegt, lässt sich durch eine zusätzliche Wasserstoffzufuhr die entsprechende Menge auf 95 % steigern. Möglich ist es in diesem Zusammenhang auch, sofern erforderlich, Kohlenstoffdioxid zusätzlich beizufügen.

Die Wasserstoffquelle kann eine Elektrolysezelle und optional einen Wasserstoffspeicher umfassen. In dieser Weise lässt sich besonders einfach Wasserstoff bereitstellen. Dabei werden an eine Reinheit des Wasserstoffs keine besonderen Anforderungen gestellt. Dies bedeutet, dass auch verunreinigter Wasserstoff eingesetzt werden kann. Verunreinigungen spielen keine Rolle, da deren Anteil unter Berücksichtigung der ohnedies nur geringen zusätzlich zugeführten Menge an Wasserstoff nicht ins Gewicht fällt.

In einer weiteren Variante umfasst die Erfindung eine Anordnung zur Gewinnung von Energie, wobei die Anordnung umfasst:
- eine erfindungsgemäße Vorrichtung;
- zumindest einen Fermenter;
- zumindest einen Wasserstoffspeicher;
- zumindest eine Verbrennungsanlage, welche CO₂ produziert;
- erste Verbindungsmittel, um Wasserstoff aus dem Wasserstoffspeicher dem zumindest einen Fermenter zuzuführen;
- optional zweite Verbindungsmittel, um CO₂ aus der zumindest einen Verbrennungsanlage dem zumindest einen Fermenter zuzuführen.

Eine entsprechende Anordnung weist den Vorteil auf, dass neben einer hohen Ausbeute an Methan eine optimale Ausnutzung von Produkten aus anderen Prozessen nutzbar ist, sodass insgesamt betrachtet eine gute Ausnutzung von Ressourcen gegeben ist. Der Fermenter kann insbesondere mit Wasserstoff beaufschlagt werden, der nicht besonders rein ist und ohne Trocknung für andere Verwendungszwecke nicht geeignet ist. In ähnlicher Weise kann CO₂ aus einer Verbrennungsanlage, beispielsweise einem Blockheizkraftwerk, zugeführt werden. Möglich ist es auch, dass Sauerstoff, der bei der Erzeugung von Wasserstoff durch Elektrolyse anfällt, dem Blockheizkraftwerk zugeführt wird.

In der Regel ist es zweckmäßig, dass stromabwärts zwischen dem Vorfermenter und dem Hauptfermenter ein weiteres Behältnis zur Neutralisation eines Substrates aus dem Vorfermenter vorgesehen ist.

Weitere Merkmale, Vorteile und Wirkungen ergeben sich aus den nachfolgend dargestellten Ausführungsbeispielen. In den Zeichnungen, auf welche dabei Bezug genommen wird, zeigen:
Fig. 1 eine Fermentationsanlage gemäß dem Stand der Technik mit einer eingebundenen erfindungsgemäßen Vorrichtung;
Fig. 2 eine Fermenteranordnung mit einer erfindungsgemäßen Vorrichtung;
Fig. 3 eine Fermenteranordnung;
Fig. 4 eine Anordnung mit einer erfindungsgemäßen Vorrichtung.

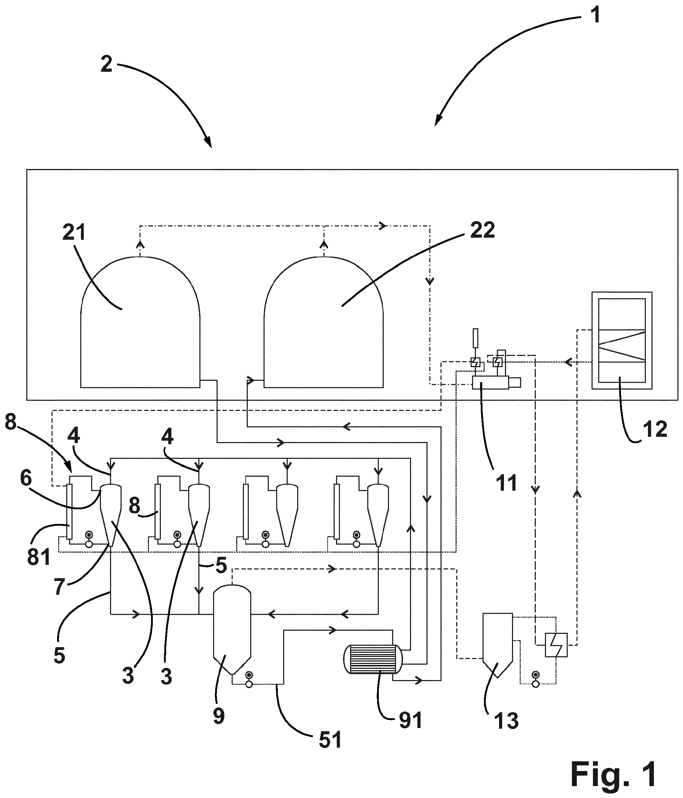

In Fig. 1 ist eine Fermenteranordnung 2 gemäß dem Stand der Technik dargestellt. Die Fermenteranordnung 2 umfasst einen Hauptfermenter 21 sowie einen nachgeschalteten Nachfermenter 22. Im Hauptfermenter 21 und im Nachfermenter 22 werden biologische Abfälle unter anaeroben Bedingungen durch Bakterien zersetzt, um Biogas zu gewinnen, das am Hauptfermenter 21 sowie am Nachfermenter 22 kopfseitig abgenommen und einem Blockheizkraftwerk 11 zugeführt wird.

Des Weiteren ist in Fig. 1 eine Vorrichtung 1 ersichtlich, die stromabwärts des Hauptfermenters 21 diesem nachgeschaltet und dem Nachfermenter 22 vorgeschaltet ist. Die Vorrichtung 1 umfasst mehrere Behältnisse 3, zum Beispiel wie in Fig. 1 vier Behältnisse 3. Grundsätzlich kann aber auch ein einzelnes Behältnis 3 oder eine beliebige Anzahl mehrerer Behältnisse 3 vorgesehen sein. Die Behältnisse 3 sind mit einem zylindrischen Rumpf ausgebildet und laufen bodenseitig konisch zu, wenngleich eine derartige Ausbildung nicht zwingend ist.

Die Behältnisse 3 stehen mit dem Hauptfermenter 21 über eine Versorgungsleitung, welchen in einzelne Zuleitungen 4 für die Behältnisse 3 mündet, in Verbindung, sodass fermentierte Biomasse aus dem Hauptfermenter 21 in die einzelnen Behältnisse 3 förderbar, insbesondere pumpbar, ist. Jedes der Behältnisse 3 ist mit einer Dampfzirkulationseinheit 8 über einen Dampfeinlass 6 sowie einen Dampfauslass 7 verbunden. Die Dampfzirkulationseinheiten 8 sind mit ersten Wärmetauschern 81 ausgebildet. An jedes der Behältnisse 3 schließt vorzugsweise bodenseitig eine Ableitung 5 an, die in einen Entspannungsbehälter 9 führt, vorzugsweise etwa etwas unterhalb des Kopfbereiches des Entspannungsbehälters 9. Vom Entspannungsbehälter 9 führt vorzugsweise bodenseitig eine weitere Ableitung 51 zu einem zweiten Wärmetauscher 91 und von dort in den Fermentationsprozess zurück zum Nachfermenter 22.

Bei einem Fermentationsprozess mit einer Vorrichtung 1 gemäß Fig. 1 wird aufzubereitende Biomasse zunächst im Hauptfermenter 21 fermentiert. Anschließend wird die verbleibende Masse der Vorrichtung 1 zugeführt, wobei zunächst über den zweiten Wärmetauscher 91 und die aus dem Entspannungsbehälter 9 abgeführte Masse ein Wärmetausch erfolgt, sodass die den einzelnen Behältnissen 3 zugeführte, bereits teilfermentierte Masse auf einer Temperatur von etwa 80 °C bis 90 °C befindlich ist. Über die Dampfzirkulationseinheiten 8 wird vorzugsweise kontinuierlich über den Dampfeinlass 6 Dampf eingebracht und über den Dampfauslass 7 abgezogen, sodass die im Behältnisse 3 verweilende Masse einer Thermohydrolyse im Temperaturbereich von bis zu 200 °C unterworfen wird. Ein Druck in den einzelnen Behältnissen 3 beträgt dabei bis zu etwa 20 bar. Dabei wird die Biomasse in den Behältnissen 3 für eine vorbestimmte Haltezeit gehalten. Nach Ablauf dieser Haltezeit wird die unter Druck stehende Biomasse in den Entspannungsbehälter 9 gegen atmosphärischen Druck entspannt. Dabei kommt es einerseits zu einer mechanischen Zerstörung der in der Biomasse enthaltenen Pflanzenfasern und andererseits zu einer spontanen Verdampfung von Wasser. Handelt es sich bei diesem verdampfenden Wasser um innerzelluläres Wasser, wird die umgebene Zellhülle aufgerissen und es kommt zu einer zusätzlichen Zerkleinerung der polymeren Substrate. Die bei diesem Entspannungsprozess entstehenden Entspannungsbrüden werden in einen Heißwasserbehälter 13 geleitet und dort auskondensiert. Dadurch kann frei gewordenen Energie wieder in Wasser gebunden werden. Das durch den Eintrag der Entspannungsbrüden erwärmte Wasser gibt die Wärmeenergie kontinuierlich wieder an eine Fernwärmestation 12 ab, was einen Energiekreislauf schließt.

Da die Thermohydrolyse in der Vorrichtung 1 ein reiner Batch-Prozess ist, eine Energieversorgung mit Dampf aus den Dampfzirkulationseinheiten 8 aber kontinuierlich erfolgt, wird die Thermohydrolyse als semikontinuierliches Verfahren betrieben. Dies bedeutet, dass für eine typische Prozessdauer von ca. 3,25 Stunden vier Aufschlussbehälter bzw. Behältnisse 3 zum Einsatz kommen. Einer der Aufschlussbehälter ist dabei in einer Aufheizphase für welche die meiste Energie benötigt wird. Zwei Aufschlussbehälter befinden sich im Haltemodus und ein Aufschlussbehälter bzw. Behältnis 3 befindet sich in einem Befüll- bzw. Entleerungs-Zyklus.

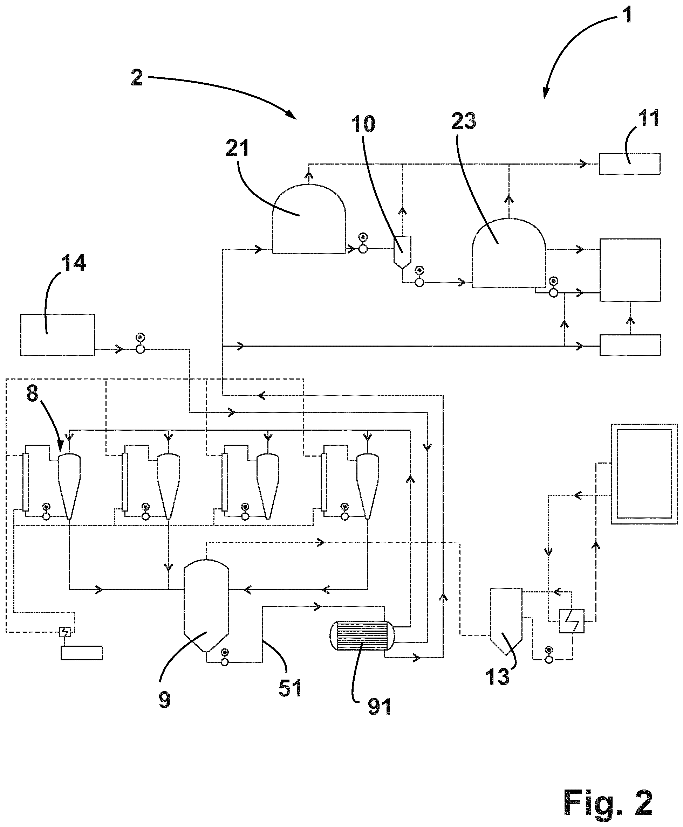

In Fig. 2 ist eine alternative Vorrichtung 1 dargestellt, die grundsätzlich ähnlich wie jene in Fig. 1 aufgebaut ist. Gemäß Fig. 2 ist jedoch die Vorrichtung 1 einem Vorfermenter 23 vorgeschaltet. In diesem Fall wird aus einem Biomassereservoir 14 noch nicht fermentierte bzw. unbehandelte Biomasse zunächst der Vorrichtung 1 zugeführt und erst dann im Vorfermenter 23 und einem Hauptfermenter 21 umgesetzt, wobei die Vorrichtung 1 in Bezug auf den Energiehaushalt weitgehend ident wie die in Fig. 1 dargestellte betrieben wird.

Eine Anlage gemäß Fig. 2 zeichnet sich allerdings nicht nur durch eine vorgeschaltete Vorrichtung 1 aus, sondern auch durch einen speziellen Vorfermenter 23 sowie einen speziellen Hauptfermenter 21, zwischen welchen ein weiteres Behältnis 10 angeordnet ist.

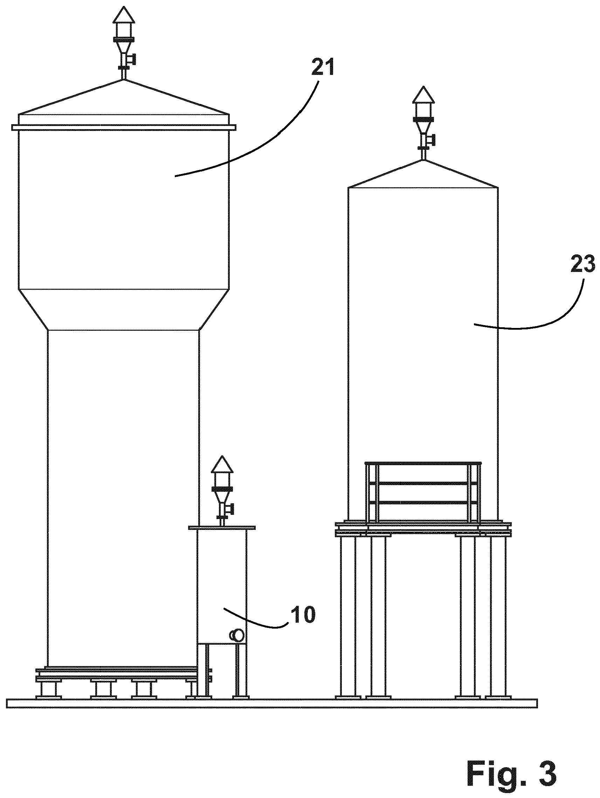

In Fig. 3 ist die Fermenteranordnung 2 aus Fig. 2 näher dargestellt. Der Vorfermenter 23 ist dabei als isolierter Doppelwand-Edelstahlbehälter ausgebildet, wobei ein Verhältnis von Durchmesser zu Höhe etwa 1:1,5 beträgt. Das in der Vorrichtung 1 durch Thermohydrolyse aufgeschlossene Substrat wird in diesen Vorfermenter 23 gepumpt, der bei einem niedrigeren pH-Wert von 5,2 bis 5,7 als der stromabwärts nachgeschaltete Hauptfermenter 21 betrieben wird, um Hydrolyse- und Acidogeneseprozesse leichter zu ermöglichen. Diese Prozesse werden im mesophilen Temperaturbereich bei ca. 38 °C gefahren, welcher durch Durchströmung des Doppelmantels des Vorfermenters 23 mit Heiz- bzw. Kühlwasser konstant gehalten wird. Um eine möglichst homogene Durchmischung des Substrats mit Bakterien zu erreichen, ist der Vorfermenter mit einem selbstansaugenden, dynamischen Begasungsgerät ausgestattet, welches das entstehende Biogas aus dem Gasraum (bzw. aus dem "Schaum"-Bereich, also dem Übergang zwischen Medium und Gasraum) des Vorfermenters 23 ansaugt und über ein Verteilsystem am Fermenterboden wieder ausstößt. Der Vorteil dieses Systems liegt darin, dass im Vorfermenter 23 eine fast turbulente Durchmischung entsteht, welche den Abbauprozess deutlich beschleunigt. Nach einer theoretischen Verweildauer von ca. 24h wird das vorfermentierte Substrat in die Neutralisation bzw. ein weiteres Behältnis 10 gepumpt. Das entstehende Biogas wird über die Gasleitung zur Gasaufbereitung geleitet.

Im weiteren Behältnis 10 wird das im Vorfermenter 23 hydrolysierte und vorversäuerte Substrat im Neutralisationsbehälter mit einer basischen Lösung oder Suspension, beispielsweise einer Natriumhydroxidlösung oder einer Kalkmilchsuspension vollautomatisch neutralisiert. Das weitere Behältnis 10 selbst verfügt über ein Nutzvolumen von mehreren Kubikmetern, ist aus Edelstahl gefertigt, nach außen hin isoliert und mit einem Propellerrührwerk ausgestattet. Die theoretische hydraulische Verweilzeit in diesem Behälter beträgt wenige Stunden.

Der dem weiteren Behältnis 10 stromabwärts folgende Hauptfermenter 21 umfasst im Wesentlichen zwei Teile: Der untere Teil ist als zylindrischer Edelstahlbehälter mit einem Doppelmantel gebildet, der an einer Außenseite isoliert ist, wohingegen ein weiterer, oberer Teil nur isoliert ist. Der untere Teil ist mit jeweils einem Verteilsystem am oberen und unteren Ende des Zylinders ausgestattet. Die Durchmischung im Hauptfermenter 21 erfolgt über eine externe Zwangszirkulation. Dies bedeutet, dass ein gewisser Volumenstrom des Ferments durch das obere Sammelsystem mittels einer Pumpe abgesaugt und an der durch das untere Verteilsystem wieder in den Fermenter eingebracht wird. Die dadurch im Hauptfermenter 21 entstehende Aufwärtsströmung sorgt für eine optimale Durchmischung im Behälter. Zusätzlich wird das vorfermentierte und neutralisierte Substrat in das untere Verteilsystem dosiert, um dadurch eine kontinuierliche Versorgung der Bakterien mit Substrat sicher zu stellen. Am Fermenterboden des Hauptfermenter 21 ist eine automatische Schlammablasseinrichtung vorgesehen über die der im Fermenter befindliche Schlammspiegel konstant gehalten wird. Der abgezogene Überschussschlamm ist sehr Mineral- und Stickstoffreich und kann entweder direkt oder nach einer Entwässerungsstufe als Düngemittel in der Landwirtschaft eingesetzt werden.

Der obere Teil des Fermenters besteht aus zwei kombinierten Einheiten. Eine Haupteinheit bildet das sogenannte Gassammelsystem und ist als ein verkehrt montierter Trichter ausgebildet, welche die aufsteigenden Gasblasen in die Mitte des Fermenters leitet. Die mit den Gasblasen mitgerissenen Bakterien sinken an der Außenseite des Gassammlers wieder zurück in den unteren Teil des Fermenters während die klare Flüssigphase am Rand des Fermenters abgezogen wird.

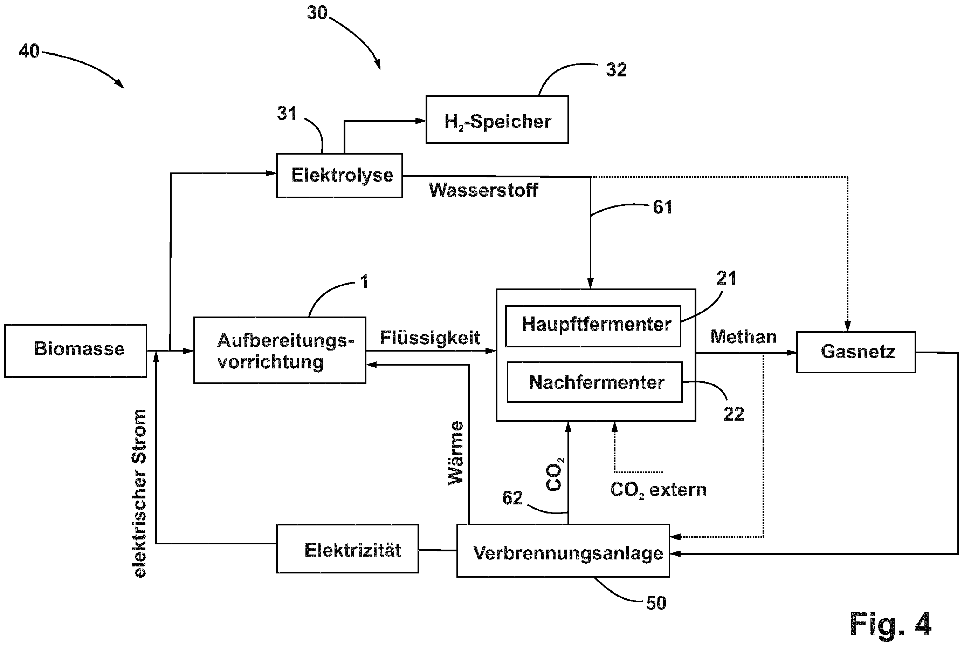

In Fig. 4 ist ein allgemeines Konzept mit einer erfindungsgemäßen Vorrichtung 1 dargestellt. Das Konzept umfasst insgesamt eine Anordnung 40, die neben der Vorrichtung 1 insbesondere auch eine Wasserstoffquelle 30 aufweist. Die Wasserstoffquelle 30 kann insbesondere eine Elektrolysezelle 31 umfassen. Möglich ist es auch, dass ein Wasserstoffspeicher 32 vorgesehen ist, um in der Elektrolysezelle 31 erzeugten Wasserstoff temporär zwischen zu speichern. Wie in Fig. 4 ersichtlich ist, kann Wasserstoff aus der Wasserstoffquelle 30 über erste Verbindungsmittel 61 der Vorrichtung 1, beispielsweise einem Hauptfermenter 21 und/oder einem Nachfermenter 22 zugeführt werden. Dadurch kann die Ausbeute an Methan erhöht werden.

Wie in Fig. 4 ersichtlich ist, umfasst die Anordnung 40 des Weiteren eine Verbrennungsanlage 50. Bei der Verbrennungsanlage 50 kann es sich beispielsweise um ein Blockheizkraftwerk handeln. Das Blockheizkraftwerk kann mit Gas betrieben werden, welches als Produkt der Biomasse erhalten wird. Die Verbrennungsanlage 50 steht über zweite Verbindungsmittel 62 ebenfalls mit der Vorrichtung 1 in Verbindung, bevorzugt wiederum mit einem Hauptfermenter 21 und/oder einem Nachfermenter 22. Dadurch ist es möglich, ebenso wie Wasserstoff auch Kohlenstoffdioxid zuzuführen und damit eine Ausbeute an Methan zu maximieren. Insgesamt kann durch die Kopplung der einzelnen Einheiten ein hocheffizienter Prozess bereitgestellt werden.

Es ist nicht erforderlich, dass Flüssigkeit aus der Vorrichtung 1 unmittelbar dem Hauptfermenter 21 nachgeführt wird. Die gewonnene Flüssigkeit kann auch separat gelagert und zu einem späteren Zeitpunkt weiterverarbeitet werden. Möglich ist es auch, dass die Flüssigkeit aus der Vorrichtung 1 transportiert und an einem anderen Ort weiterarbeitet wird. Somit erlaubt die Anordnung 40 die Erzeugung von Biogas, aber auch einen Betrieb, bei dem ein Teil oder gegebenenfalls zumindest temporär auch die gesamte Flüssigkeit gelagert und/oder aus dem Prozess abgezogen wird.

Möglich ist es auch, dass mehrere Vorrichtungen 1 seriell oder parallel, beispielsweise in einem Ring, geschaltet sind, wenn es zur Erreichung bestimmter Kapazitäten notwendig ist.

## Patentansprüche

1. Vorrichtung (1) zur Aufbereitung von Biomasse für einen Fermenter, umfassend zumindest ein Behältnis (3), zumindest eine Zuleitung (4) und zumindest eine Ableitung (5), wobei Biomasse über die Zuleitung (4) in das zumindest eine Behältnis (3) führbar und über die zumindest eine Ableitung (5) aus diesem abführbar ist, **dadurch gekennzeichnet, dass** das zumindest eine Behältnis (3) mit zumindest einem Dampfeinlass (6) und zumindest einem Dampfauslass (7) ausgebildet ist und zumindest eine nebengeordnete Dampfzirkulationseinheit (8) vorgesehen ist, welche über den zumindest einen Dampfeinlass (6) und den zumindest einen Dampfauslass (7) mit dem Behältnis (3) in Verbindung steht und über welche Dampf im Behältnis (3) zirkulierbar ist, um die Biomasse im Behältnis (3) unter erhöhtem Druck und bei erhöhter Temperatur zu behandeln, und dass dem Behältnis (3) stromabwärts ein Entspannungsbehälter (9) nachgeschaltet ist, in welchem die im Behältnis (3) behandelte Biomasse gegen Atmosphärendruck entspannbar ist.

2. Vorrichtung (1) nach Anspruch 1, **dadurch gekennzeichnet, dass** die Dampfzirkulationseinheit (8) einen ersten Wärmetauscher (81), insbesondere einen Rohrbündelwärmetauscher, umfasst.

3. Vorrichtung (1) nach Anspruch 1 oder 2, **dadurch gekennzeichnet, dass** zumindest ein zweiter Wärmetauscher (91) vorgesehen ist, welcher dem Entspannungsbehälter (9) nachgeschaltet ist und mit der welcher mit der Zuleitung (4) in Wirkverbindung steht, sodass die Biomasse vor deren Zuführung zum Behältnis (3) durch Wärmetausch vorwärmbar ist.

4. Vorrichtung (1) nach einem der Ansprüche 1 bis 3, **dadurch gekennzeichnet, dass** ein oder mehrere Container vorgesehen sind, welche die Vorrichtung (1) zumindest weitgehend außenseitig abschließen.

5. Verfahren zur Aufbereitung von Biomasse für einen Fermenter mit einer Vorrichtung (1) nach einem der Ansprüche 1 bis 4, **dadurch gekennzeichnet, dass** in zumindest einem Behältnis (3) Dampf kontinuierlich zirkuliert und die Biomasse für eine vorbestimmte Zeit im Behältnis (3) gehalten und anschließend dem Entspannungsbehälter (9) zugeführt wird.

6. Verfahren nach Anspruch 5, **dadurch gekennzeichnet, dass** die Biomasse in den Behältnissen (3) einem Zyklus mit Befüllung, Aufheizen auf eine Temperatur und Halten bei dieser Temperatur unterworfen wird, wobei die Zyklen in den Behältnissen (3) zeitversetzt ablaufen können.

7. Verfahren nach Anspruch 5 oder 6, **dadurch gekennzeichnet, dass** die die Biomasse vor der Zuführung zum Behältnis (3) erwärmt wird.

8. Verfahren nach Anspruch 7, **dadurch gekennzeichnet, dass** im Entspannungsbehälter (9) freiwerdende Energie zur Vorwärmung der Biomasse genutzt wird.

9. Fließfähige Masse, erhältlich durch ein Verfahren nach einem der Ansprüche 5 bis 8.

10. Fermenteranordnung (2), umfassend einen Hauptfermenter (21) und optional einen nachgeschalteten Nachfermenter (22), **dadurch gekennzeichnet, dass** eine Vorrichtung (1) nach einem der Ansprüche 1 bis 4 dem Hauptfermenter (21) vorgeschaltet oder stromabwärts vor dem Nachfermenter (22) dem Hauptfermenter (21) nachgeschaltet ist.

11. Fermenteranordnung (2) mit zumindest einem Fermenter, **dadurch gekennzeichnet, dass** eine Vorrichtung (1) nach einem der Ansprüche 1 bis 4 vorgesehen ist und der zumindest eine Fermenter mit einer Wasserstoffquelle (30) in Verbindung steht und/oder mit dieser in Verbindung bringbar ist, sodass Wasserstoff in den zumindest einen Fermenter einbringbar ist.

12. Fermenteranordnung (2) nach Anspruch 11, **dadurch gekennzeichnet, dass** die Wasserstoffquelle (30) eine Elektrolysezelle (31) und optional einen Wasserstoffspeicher (32) umfasst.

13. Anordnung (40) zur Gewinnung von Energie, **dadurch gekennzeichnet, dass** die Anordnung (40) umfasst:
- eine Vorrichtung (1) nach einem der Ansprüche 1 bis 4;
- zumindest einen Fermenter;
- zumindest einen Wasserstoffspeicher (32);
- zumindest eine Verbrennungsanlage (50), welche CO₂ produziert;
- erste Verbindungsmittel (61), um Wasserstoff aus dem Wasserstoffspeicher (32) dem zumindest einen Fermenter zuzuführen;
- optional zweite Verbindungsmittel (62), um CO₂ aus der zumindest einen Verbrennungsanlage (50) dem zumindest einen Fermenter zuzuführen.

14. Fermenteranordnung (2), umfassend mehrere in Serie geschaltete Fermenter zur Aufbereitung von Biomasse, insbesondere Abfällen und/oder Müll aus Biomasse, **dadurch gekennzeichnet, dass** ein Vorfermenter (23) und ein Hauptfermenter (21) vorgesehen sind und im und/oder am Vorfermenter (23) und Hauptfermenter (21) Mittel zur Umwälzung der aufzubereitenden Biomasse vorgesehen sind.

15. Fermenteranordnung (2) nach Anspruch 14, **dadurch gekennzeichnet, dass** stromabwärts zwischen dem Vorfermenter (23) und dem Hauptfermenter (21) ein weiteres Behältnis (10) zur Neutralisation eines Substrates aus dem Vorfermenter (23) vorgesehen ist.
